(19) Europäisches Patentamt
European Patent Office
Office européen des brevets

(11) Veröffentlichungsnummer: **0 326 027 B1**

(12) **EUROPÄISCHE PATENTSCHRIFT**

(45) Veröffentlichungstag der Patentschrift: **23.12.92**

(51) Int. Cl.5: **C07C 57/30**, C07C 51/44

(21) Anmeldenummer: **89100912.8**

(22) Anmeldetag: **20.01.89**

# (54) Verfahren zur Reinigung von 2-(4-Isobutylphenyl)-propionsäure.

(30) Priorität: **29.01.88 DE 3802619**

(43) Veröffentlichungstag der Anmeldung:
**02.08.89 Patentblatt 89/31**

(45) Bekanntmachung des Hinweises auf die Patenterteilung:
**23.12.92 Patentblatt 92/52**

(84) Benannte Vertragsstaaten:
**AT BE CH DE ES FR GB GR IT LI LU NL SE**

(56) Entgegenhaltungen:
**DE-A- 2 557 011**
**GB-A- 1 552 202**
**US-A- 3 344 045**

(73) Patentinhaber: **HOECHST AKTIENGESELLSCHAFT**
**Postfach 80 03 20**
**W-6230 Frankfurt am Main 80(DE)**

Patentinhaber: **HOECHST CELANESE CORPORATION**
**Route 202-206 North**
**Somerville, N.J. 08876(US)**

(72) Erfinder: **Rittner, Siegbert, Dr.**
**Kornblumenweg 5**
**W-6082 Mörfelden-Walldorf(DE)**
Erfinder: **Schmidt, Adolf, Dr.**
**Hainerweg 23**
**W-6238 Hofheim am Taunus(DE)**
Erfinder: **Wheeler, Larry O., Dr.**
**11122 Jackson Terrace**
**Corpus Christi, TX 78410(US)**
Erfinder: **Moss, Gary L.**
**1113 Mulholland Drive**
**Corpus Christi, TX 78410(US)**
Erfinder: **Zey, Edward G., Dr.**
**522 Evergreen**
**Corpus Christi, TX 78412(US)**

(74) Vertreter: **Meyer-Dulheuer, Karl-Hermann, Dr. et al**
**HOECHST Aktiengesellschaft Postfach 3540**
**W-6200 Wiesbaden(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**Beschreibung**

Die vorliegende Erfindung betrifft ein Verfahren zur Reinigung von 2-(4-Isobutylphenyl)-propionsäure (nachfolgend 2,4-Säure bezeichnet). Die genannte Verbindung findet vielfältige Verwendung z.B. als Analgetikum, Antipyretikum oder Antirheumatikum und als Zwischenprodukt für viele weitere Stoffe. In Form von Aminsalzen zeigen wäßrige Lösungen der 2,4-Säure ein gutes Korrosionsschutzverhalten bei der spanabhebenden Metallbearbeitung.

Es gibt zahlreiche Möglichkeiten, die 2,4-Säure zu synthetisieren. Eine Möglichkeit (Methode A), die Verbindung herzustellen, ist die Carbonylierung von 1-(4-Isobutylphenyl)-ethanol mit Kohlenmonoxid in Gegenwart von Triphenylphosphin und gegebenenfalls in Gegenwart eines Übergangsmetallhalogenids,wie z.B. in der EP 0284 310 beschrieben. Bei optimierter Verfahrensdurchführung enthält das resultierende zu reinigende Reaktionsgemisch ca. 85 % bis 93 % 2,4-Säure. Neben der 2,4-Säure liegen in dem Reaktionsgemisch noch ca. 40 organische Verunreinigungen sowie Triphenylphosphin und Triphenylphosphinoxid vor. Um aus diesem Reaktionsgemisch, das zwischen 65 °C und 70 °C schmilzt, eine gereinigte, als Arzneimittel verwendbare 2,4-Säure zu erhalten, müssen einerseits die organischen Verunreinigungen und andererseits das Triphenylphosphin und Triphenylphosphinoxid abgetrennt oder auf eine akzeptierbare Menge reduziert werden, wobei in dem gereinigten Produkt Triphenylphosphin und Triphenylphosphinoxid in einer Konzentration von weniger als 10 ppm vorliegen sollte.

Allein die Abtrennung organischer Nebenprodukte aus einem 2,4-Säure-Reaktionsgemisch ohne Triphenylphosphin und Triphenylphosphinoxid ist schon sehr aufwendig und erfordert, wie aus der Rumänischen Patentschrift 79 345 hervorgeht, mehrere Verfahrensschritte. So muß beispielsweise zunächst die 2,4-Säure in ein wasserlösliches Salz, z.B. mit Natronlauge ins Natriumsalz, überführt werden, dieses durch Extraktion mit Methylenchlorid von Neutralstoffen befreit, die wäßrige Raffinatlösung mit Kohle entfärbt, danach die Carbonsäure mit einer geeigneten Säure, Salzsäure, in Freiheit gesetzt und schließlich aus Wasser/Methanol umkristallisiert und getrocknet werden.

In der deutschen Offenlegungsschrift 25 57 011 wird in Beispiel 11 beschrieben, wie das Reaktionsgemisch von Phenylethylchlorid und Na-Methylat destillativ aufgearbeitet wird. In dem britischen Patent 1552202 wird in den Beispielen offenbart, wie Phenylalkylcarboxylsäuren durch Umkristallisation bzw. Phenylalkylcarboxylsäureester durch Destillation aufgearbeitet werden können.

In einer anderen Patentschrift (GB 971 700) wird beschrieben, daß die 2,4-Säure aus einem Reaktionsgemisch erst nach 3-maliger Umkristallisation in reiner Form vorliegt. Auch bei einem neueren, sehr aufwendigen Verfahren, wie es in der EP-A 170 147 beschrieben ist, wird als Reinigungsmethode für die 2,4-Säure die Umkristallisation gewählt.

Besonders problematisch wird die Trennaufgabe, wenn - wie z.B. bei der oben beschriebenen Methode A - Triphenylphosphin im Reaktionsgemisch vorliegt. Triphenylphosphin bildet als Base mit der 2,4-Säure und anderen Säuren des Reaktionsgemisches ein bei Raumtemperatur stabiles Addukt vom Typ der nachstehenden Formel

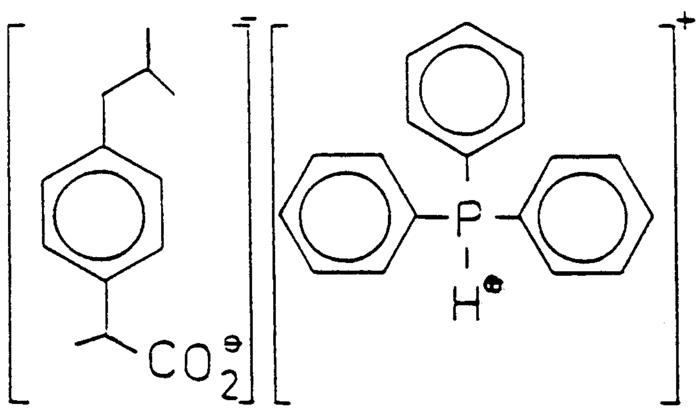

das - wie eigene Versuche zeigten - weder durch Extraktion noch durch mehrmalige Umkristallisation aus Lösemitteln abgetrennt werden kann (siehe Vergleichsbeispiel). Auch andere Trennverfahren wie die Adsorption an Harzen oder Ionenaustauschern führen nicht zum Ziel einer ausreichenden Abtrennung aller Verunreinigungen.

Bei der Suche nach einem geeigneten Trennverfahren zur Reinigung von Reaktionsgemischen zur

Gewinnung von 2,4-Säure wurde nun gefunden, daß diese Aufgabe durch eine Vakuumrektifikation gelöst werden kann. Dies ist außerordentlich überraschend, weil bis zur Lösung der genannten Aufgabe durch die vorliegende Erfindung die Reinigung der bei 74,8°C schmelzenden 2,4-Säure durch Destillation oder Rektifikation offensichtlich als undurchführbar galt. In der Literatur sind weder Dampfdruckwerte noch der Siedepunkt für die oberhalb des Schmelzpunktes von 74,8°C vorliegende ölig-viskose flüssige Säure bekannt. Der relativ hohe Schmelzpunkt der Säure macht es verständlich, daß die Chancen einer Reinigung vorwiegend in der Kristallisation aus verschiedenen Lösemitteln gesucht und - bei Abwesenheit von Triphenylphosphin - auch gefunden wurden, wie z.B. in den vorerwähnten Patentschriften beschrieben. Weiterhin erschien eine rektifikative Reinigung, bedingt durch die Instabilität der 2,4-Säure bei hohen Temperaturen, offensichtlich von vornherein wenig erfolgversprechend, zumal wenn sich im Rohsäuregemisch noch andere reaktionsfähige Komponenten wie Triphenylphosphin, Triphenylphosphinoxid, Ketone, Styrolderivate, Alkohole und Basen befinden können, die eine tiefschwarze Verfärbung bis zur Verteerung oder Verharzung des Gemisches verursachen. Nicht zuletzt mußte man darüber hinaus vermuten, daß die 2,4-Säure mit einer oder mehreren organischen Verunreinigungen azeotrope Gemische bildet.

Erfindungsgegenstand ist demzufolge ein Verfahren zur Reinigung von 2-(4-Isobutylphenyl)-propionsäure aus Gemischen, wie sie bei der Herstellung der 2-(4-Isobutylphenyl)-propionsäure anfallen, dadurch gekennzeichnet, daß die Gemische einer Vakuumrektifikation unterworfen werden. Das erfindungsgemäße Verfahren hat gegenüber den Verfahren gemäß dem Stand der Technik den Vorteil, daß die 2,4-Säure von zahlreichen gleichzeitig vorhandenen Verunreinigungen, insbesondere auch von Triphenylphophin und Triphenylphosphinoxid in einer einzigen verfahrenstechnischen Grundoperation befreit werden kann, wobei praktisch kein Abwasser und keine Abluft anfallen.

Zur Durchführung des erfindungsgemäßen Verfahrens können unterschiedliche Kolonnentypen eingesetzt werden. Kolonnen mit Gewebepackungen und Kolonnen, die einen vergleichbaren Druckverlust wie Kolonnen mit Gewebepackungen aufweisen, sind für das erfindungsgemäße Verfahren besonders geeignet, weil bei ihnen ein im Vergleich zu anderen Kolonnen geringer Druckverlust auftritt. Unter Druckverlust ist in diesem Zusammenhang die Differenz zwischen dem Druck im Bereich des Sumpfes und des Kopfes der Kolonne gemeint. Ein geringer Druckverlust bedingt eine geringe Temperaturdifferenz zwischen Sumpf und Kopf einer Kolonne und ist vonnöten, weil die Sumpftemperatur bei der Rektifikation der 2,4-Säure eine bestimmte Obergrenze nicht überschreiten sollte, da die 2,4-Säure sich bei hoher Temperatur zersetzt. Die Zersetzlichkeit der 2,4-Säure wird durch Figur 1 verdeutlicht, in der in Abhängigkeit von der Erhitzungsdauer der 2,4-Säure der jeweilige Schmelzpunkt - dessen Erniedrigung ein Maß für Verunreinigungen ist - aufgetragen ist. Man erkennt, daß bereits bei 250°C der Schmelzpunkt der jeweiligen Probe bereits nach kurzer Erhitzungszeit sinkt, was auf die Zersetzung der 2,4-Säure zurückzuführen ist. Die Zersetzlichkeit der 2,4-Säure kann durch Verunreinigungen noch gesteigert werden. Aus diesem Grund ist die Maximaltemperatur, bei der das erfindungsgemäße Verfahren noch sinnvoll eingesetzt werden kann, von der Gemischzusammensetzung abhängig. Zur Reinigung des gemäß Methode A anfallenden Reaktionsgemisches sollte bei Benutzung einer herkömmlichen Rektifikationsapparatur (Blasenrektifikationsapparatur), wie z.B. in Fig. 2 abgebildet, die Sumpftemperatur deshalb bei der Rektifikation vorzugsweise nicht über ca. 250°C liegen, besonders bevorzugt nicht über ca. 230°C, insbesondere nicht über 210°C.

Bei Benutzung moderner Rektifikationsapparaturen, wie z.B. solchen, die mit Dünnschicht- oder Fallfilmverdampfern ausgerüstet sind, lassen sich sehr kurze Verweilzeiten des zu rektifizierenden Stoffes erreichen, und somit sind bei derartigen Rektifikationsapparaturen höhere Sumpftemperaturen möglich, ohne daß größere Produktverluste auftreten. Die Sumpftemperatur bei Benutzung der zuletzt genannten Rektifikationsapparatur sollte 280°C nicht überschreiten.

Die Siedetemperatur im Sumpf und die Dampftemperatur im Bereich des Kopfes der Kolonne sind durch den Dampfdruck gegeben. Zum Beispiel bei einem Dampfdruck von 10 hPa im Kolonnenkopf und von 13 hPa im Sumpf geht die reine 2,4-Säure bei 178°C innerhalb eines Temperaturbereiches von 0,1°C überkopf, während im Sumpf - bedingt durch den höheren Druck und den Gehalt an Verunreinigungen - die Siedetemperatur zwischen 190 und 220°C variiert. Es ist vorteilhaft, den Dampfdruck im Bereich des Sumpfes niedriger als ca. 60 hPa zu halten, um eine Siedetemperatur im Sumpf von 250°C nicht zu überschreiten.

Die Anzahl der theoretischen Böden der Trennkolonne kann in einem weiten Bereich variiert werden. Zu bevorzugen ist eine Anzahl der theoretischen Böden zwischen ca. 10 und 150, besonders bevorzugt zwischen ca. 25 und 70.

Die für das erfindungsgemäße Reinigungsverfahren geeigneten Rektifikationsvorrichtungen können unterschiedlich aufgebaut sein. Eine beispielhafte Rektifikationseinrichtung im Labormaßstab zeigt die Figur 2. In der Blase (1) der Kolonne wird das die 2,4-Säure enthaltende Gemisch erhitzt. Anstelle der Blase kann die Vorrichtung auch z.B. einen Dünnschicht- oder oder Fallfilmverdampfer enthalten. Am Kopf (2) der

Kolonne können leichtsiedende Verunreinigungen abdestilliert werden. Unterhalb des Kondensators, der z.B. eine Kühlschlange (3) sein kann, wird das Kondensat in dem Flüssigkeitsteiler (4), mit dem auch das Rücklaufverhältnis einstellbar ist, aufgefangen. Die Vorlage (5), in der verschiedene Destillat-Fraktionen und das Reinprodukt gesammelt werden, ist vorteilhafterweise auf ca. 80 ° C zu temperieren, damit das Produkt nicht erstarrt. Der zur Rektifikation nötige Unterdruck wird durch geeignete Vakuumpumpen (6) erzeugt. Die Trennkolonne (7) ist mit Gewebepackungen gepackt und entwickelt eine Trennleistung von maximal 25 theoretischen Trennstufen.

Es ist zweckmäßig, die Rektifikation unter Inertgas durchzuführen, das in der bevorzugten Destillationseinrichtung z.B. über eine Siedekapillare (8) in den Sumpf der Kolonne eingeleitet werden kann. Es sind für diesen Zweck verschiedene inerte Gase geeignet. Bevorzugt sind Stickstoff, Argon und Kohlendioxid, besonders bevorzugt ist Stickstoff. Es kann auch zweckmäßig sein, die rohe 2,4-Säure vor der Rektifikation zunächst mit Wasser zu extrahieren, um wasserlösliche Verunreinigungen wie Chloride, Phosphate, Metallsalze und ähnliches zu entfernen.

Prinzipiell kann die erfindungsgemäße Rektifikation mit Hilfe einer oder mehrerer Kolonnen sowohl diskontinuierlich, d.h. durch Abnahme einzelner Fraktionen, als auch kontinuierlich durchgeführt werden. Wird eine kontinuierliche Ein-Kolonnen-Destillation durchgeführt, dann kann die Kolonne vorzugsweise so eingesetzt werden, daß das Reinprodukt etwa im mittleren Bereich der Säule dampfförmig seitlich abgezogen wird, während die Säule vorzugsweise unter vollständigem Rückfluß betrieben wird. Bei der kontinuierlichen Rektifikation können vor der Abtrennung der 2,4-Säure sowohl die leichtsiedenden oder auch die schwer siedenden Stoffe zuerst abgetrennt werden, wenn dafür die für das jeweilige Reaktionsgemisch kritische Temperatur-Stabilitätsgrenze nicht überschritten wird.

Das erfindungsgemäße Verfahren ist an sich zur Aufarbeitung beliebiger 2,4-Säure-haltiger Gemische geeignet. Besondere Bedeutung besitzt es allerdings bei der Aufarbeitung von Gemischen, die bei der Synthese von 2,4-Säure anfallen, insbesondere für solche Reaktionsgemische, die bei der oben beschriebenen Methode A entstehen.

Das erfindungsgemäße Verfahren ist geeignet, die aus den Herstellungsverfahren gemäß Methode A anfallende Rohsäure ohne Vorreinigung von allen Verunreinigungen zu befreien. Es kann aber auch sinnvoll sein, rektifikativ nur einen Teil der Verunreinigungen abzutrennen und die restlichen Verunreinigungen durch eine oder mehrere Reinigungsverfahren abzutrennen. Besonders geeignet sind hierzu die Kristallisation aus Lösemitteln und die hilfsstoffreie Schmelzkristallisation. Insbesondere kann es sinnvoll sein, vor der Rektifikation mindestens eine Schmelzkristallisation durchzuführen. Dabei wird die Rohsäureschmelze durch Abkühlung in einen Blockkristallisat umgewandelt, aus dem die stark verunreinigte Restschmelze ausgeschieden und das Kristallisat nach Aufschmelzen der erfindungsgemäßen Rektifikation unterworfen wird. Durch die genannten Verfahrenskombinationen kann die erfindungsgemäße Rektifikation mit einer Trennkolonne mit kleinerer theoretischer Bodenzahl durchgeführt werden. Durch die nachfolgenden Ausführungsbeispiele soll die Erfindung näher erläutert werden.

**Beispiel 1**

In einer Apparatur zur fraktionierten Rektifikation wie in Fig. 2 dargestellt wurden 500 g eines ca. 90 %igen 2,4-Säure-Gemisches der folgenden Zusammensetzung eingesetzt.

Tabelle 1

| Verbindung | Gew.-% |
|---|---|
| 2-(4-Isobutylphenyl)-propionsäure | 88,77 |
| Triphenylphosphin | 0,18 |
| Isobutylphenylethan | 1,90 |
| 4-Isobutylbenzol | 0,02 |
| 4-Isobutylstyrol | 0,15 |
| 4-Isobutylacetophenon | 0,78 |
| 1-(4-Isobutylphenyl)-ethanol | 0,08 |
| 1-(4-Isobutylphenyl)-chlorethan | 0,59 |
| 2-(4-Isobutylphenyl)-propionsäure-ethylester | 0,05 |
| 2-(3-Isobutylphenyl)-propionsäure | 1,10 |
| 3-(4-Isobutylphenyl)-propionsäure | 1,90 |
| Leichtsieder mit einem Durchschnittsmolgew. v. 178 | 1,80 |
| Schwersieder mit einem Durchschnittsmolgew. v. 320 | 1,20 |
| Methylethylketon | 0,48 |
| übrige nicht identifizierte Verunreinigungen ca. | 1,0 |

Fig. 3 zeigt ein Gaschromatogramm aller Verunreinigungen in dem Gemisch.

Das die genannten Verunreinigungen enthaltende Gemisch wurde in Gegenwart von Stickstoff bei einem Druck von 10 hPa und bei einer Sumpftemperatur von anfänglich 150 bis schließlich 230 °C mit wechselnden Rücklaufverhältnissen fraktioniert destilliert. Hierbei wurden die folgenden Destillat-Fraktionen abgenommen:

1) 20 g Vorlauf als gelbe Flüssigkeit
2) 158 g Zwischenlauf mit einem 2,4-Säuregehalt von 94 - 98 %
3) 300 g Hauptlauf.

Als Rückstand verblieben im Kolben 22 g. Der Hauptlauf hatte die folgende Zusammensetzung:

| Verbindungen | Gew.-% |
|---|---|
| 2-(4-Isobutylphenyl)-propionsäure | 99,5 |
| Triphenylphosphin | 0,00005 |
| Übrige nicht phosphororganische Verunreinigungen | 0,5 |

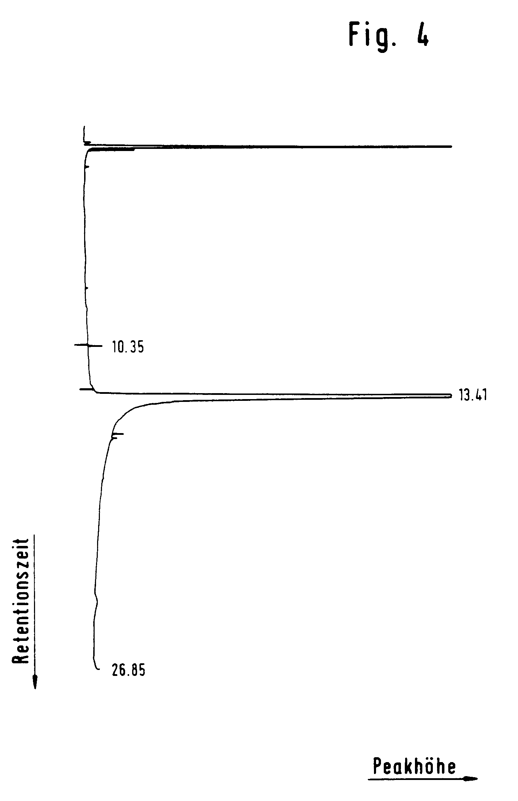

Fig. 4 zeigt ein Gaschromatogramm der Hauptfraktion.

**Beispiel 2**

Mit Hilfe der in Beispiel 1 beschriebenen Rektifikationsapparatur wurde das dort beschriebene ca. 90 %ige 2,4-Säure-Gemisch einer zweistufigen Rektifikation unterworfen. Dabei wurde beim ersten Destillationsdurchgang nach Abtrennung des Vorlaufs der Schwerpunkt nur auf die Abtrennung des Sumpfes gelegt, in dem sich die kritische Triphenylphosphin-Verunreinigung anreichert. In einem zweiten Rektifikationsdurchgang wurde dann der ca. 97 % 2,4-Säure enthaltende Hauptlauf der ersten Rektifikation nochmals rektifiziert, wobei die Sumpftemperatur bei ca. 200 °C lag. Hierbei wurden aus 942 g Einsatzprodukt die folgenden Fraktionen gewonnen:

1) 245 g Zwischenlauf mit einem Gehalt an 2,4-Säure bis 98,8 %
2) 640 g Hauptlauf
3) 57 g Destillationsrückstand.

Der Hauptlauf hatte die folgende Zusammensetzung:

| Verbindungen | Gew.-% |
|---|---|
| 2-(4-Isobutylphenyl)-propionsäure<br>Triphenylphosphin<br>Übrige nicht phosphororganische Verunreinigungen | 99,5<br>0,00005<br>0,5 |

**Beispiel 3**

In einem Röhrenkristallisator, dessen Kühl-/Heizmantel an einen Thermostaten angeschlossen ist, wurde das wie in Beispiel 1 zusammengesetzte ca. 90 %ige 2,4-Säuregemisch als Schmelze vorgelegt. Man kühlte die Schmelze mit einem Erstarrungspunkt von 58° C innerhalb von 10 Stunden auf 40° C ab, wobei sich im Apparat ein kompaktes Kristallisat abschied. Danach wurde der im Kristallisator verbliebene dunkelbraune Flüssiganteil abgelassen und die so gewonnenen hellen Kristalle aufgeschmolzen und der erfindungsgemäßen Rektifikation unterworfen. Aus 500 g 90 %igem Einsatzprodukt wurden 440 g Kristallisat mit einer Reinheit an Säure von 98,5 % gewonnen.

Die auf diese Weise vorgereinigte 2,4-Säure wurde nun der erfindungsgemäßen Rektifikation bis zu einer Sumpftemperatur von 220° C unterworfen. Hierbei wurden rund 370 g einer Hauptfraktion mit der folgenden Zusammensetzung erhalten:

| Verbindungen | Gew.-% |
|---|---|
| 2-(4-Isobutylphenyl)-propionsäure<br>Triphenylphosphin<br>Übrige nicht phosphororganische Verunreinigungen | 99,6<br>0,00005<br>0,4 |

**Vergleichsbeispiel 1**

In einem 250 ml Glaskolben mit Rückflußkühler wurden 20 g 2,4-Säure, die 0,4 % Triphenylphosphin enthielten, in 50 ml n-Hexan bei Rückflußtemperatur des Lösemittels aufgelöst. Danach wurde die Lösung auf Raumtemperatur abgekühlt, die ausgefallene Säure abfiltriert mit 20 ml kaltem n-Hexan gewaschen und im Exsikkator getrocknet. Die Anlayse der umkristallisierten Säure zeigte, daß der Triphenylphosphin-Gehalt unverändert geblieben war. Die so gewonnene 2,4-Säure wurde danach noch ein zweites, drittes und ein viertes Mal aus n-Hexan umkristallisiert, ohne daß der Triphenylphosphin-Gehalt verändert werden konnte.

**Patentansprüche**

**1.** Verfahren zur Reinigung von 2-(4-Isobutylphenyl)-propionsäure aus Gemischen, die bei der Herstellung der 2-(4-Isobutylphenyl)-propionsäure anfallen, dadurch gekennzeichnet, daß die Gemische einer Vakuumrektifikation unterworfen werden bei Sümpftemperatüren bis 280° C.

**2.** Verfahren nach Anspruch 1, dadurch gekennzeichnet, daß die Rektifikation mit Kolonnen, die mit Gewebepackungen ausgerüstet sind oder mit Kolonnen, die einen vergleichbar kleinen Druckverlust aufweisen, durchgeführt wird.

**3.** Verfahren nach Anspruch 1 oder 2, dadurch gekennzeichnet, daß die Rektifikation unter Benutzung einer Blasenrektifikationsapparatur bei Sumpftemperaturen unterhalb von ca. 250° C durchgeführt wird.

**4.** Verfahren nach Anspruch 1 oder 2, dadurch gekennzeichnet, daß die Rektifikation unter Benutzung einer mit einem Dünnschicht- oder Fallfilmverdampfer ausgerüsteten Rektifikationsapparatur bei Sumpftemperaturen unterhalb von ca. 280° C durchgeführt wird.

**5.** Verfahren nach einem oder mehreren der Ansprüche 1 - 4, dadurch gekennzeichnet, daß die Trennkolonne zwischen ca. 10 und 150 theoretische Böden aufweist.

**6.** Verfahren nach einem oder mehreren der Ansprüche 1 - 5, dadurch gekennzeichnet, daß die

Rektifikation in Gegenwart eines Inertgases durchgeführt wird.

7. Verfahren nach einem oder mehreren der Ansprüche 1 - 6, dadurch gekennzeichnet, daß die Rektifikation mit einer oder mehreren Kolonnen diskontinuierlich durchgeführt wird.

8. Verfahren nach einem oder mehreren der Ansprüche 1 - 6, dadurch gekennzeichnet, daß die Rektifikation kontinuierlich mit einer oder mehreren Kolonnen durchgeführt wird.

9. Verfahren nach einem oder mehreren der Ansprüche 1 - 8, dadurch gekennzeichnet, daß die 2-(4-Isobutylphenyl)-propionsäure aus einem Gemisch, das bei der Carbonylierung von 1-(4-Isobutylphenyl)-ethanol mit Kohlenmonoxid in Gegenwart von Triphenylphosphin und gegebenenfalls in Gegenwart eines Übergangsmetallhalogenids anfällt, isoliert wird.

10. Verfahren nach einem oder mehreren der Ansprüche 1 - 9, dadurch gekennzeichnet, daß die 2-(4-Isobutylphenyl)-propionsäure durch die Rektifikation von allen Verunreinigungen befreit wird.

11. Verfahren nach einem oder mehreren der Ansprüche 1 - 9, dadurch gekennzeichnet, daß zur Rektifikation ein Gemisch eingesetzt wird, das zuvor durch mindestens eine Schmelzkristallisation von einem Teil der Verunreinigungen befreit worden ist.

12. Verfahren nach einem oder mehreren der Ansprüche 1 - 9, dadurch gekennzeichnet, daß rektifikativ nur ein Teil der Verunreinigungen abgetrennt wird und zur Abtrennung der restlichen Verunreinigungen eine Kristallisation aus Lösemitteln und/oder eine Schmelzkristallisation durchgeführt wird.

## Claims

1. A process for the purification of 2-(4-isobutylphenyl)-propionic acid from mixtures obtained in the preparation of 2-(4-isobutylphenyl)-propionic acid, which comprises subjecting the mixtures to a vacuum rectification at bottom temperatures of up to 280 °C.

2. The process as claimed in claim 1, wherein the rectification is carried out by means of columns provided with fabric packing, or columns having a comparably small pressure drop.

3. The process as claimed in claim 1 or 2, wherein the rectification is carried out using a still rectification apparatus at bottom temperatures below about 250 °C.

4. The process as claimed in claim 1 or 2, wherein the rectification is carried out using a rectification apparatus fitted with a thin-layer evaporator or falling-film evaporator, at bottom temperatures below about 280 °C.

5. The process as claimed in one or more of claims 1-4, wherein the number of theoretical plates in the separation column is between about 10 and 150.

6. The process as claimed in one or more of claims 1-5, wherein the rectification is carried out in the presence of an inert gas.

7. The process as claimed in one or more of claims 1-6, wherein the rectification is carried out discontinuously, using one or more columns.

8. The process as claimed in one or more of claims 1-6, wherein the rectification is carried out continuously, using one or more columns.

9. The process as claimed in one or more of claims 1-8, wherein the 2-(4-isobutylphenyl)-propionic acid is isolated from a mixture obtained in the carbonylation of 1-(4-isobutylphenyl)-ethanol with carbon monoxide in the presence of triphenylphosphine and, if appropriate, in the presence of a transition metal halide.

10. The process as claimed in one or more of claims 1-9, wherein the 2-(4-isobutylphenyl)-propionic acid is

freed of all impurities by the rectification.

11. The process as claimed in one or more of claims 1-9, wherein a mixture is used for rectification, from which a part of the impurities has been removed beforehand by at least one melt crystallization.

12. The process as claimed in one or more of claims 1-9, wherein only a part of the impurities is separated off by rectification and, for separating off the remaining impurities, a crystallization from solvents and/or a melt crystallization are carried out.

## Revendications

1. Procédé pour la purification de l'acide 2-(4-isobutylphényl)-propionique à partir de mélanges qui sont produits lors de la préparation de l'acide 2-(4-isobutylphényl)-propionique, caractérisé en ce que l'on soumet les mélanges à une rectification sous vide à des températures au bas de la colonne allant jusqu'à 280 ° C.

2. Procédé selon la revendication 1, caractérisé en ce que la rectification est effectuée avec des colonnes qui sont munies de garnissages en tissu, ou avec des colonnes qui présentent une faible chute de pression comparable.

3. Procédé selon la revendication 1 ou 2, caractérisé en ce que la rectification est effectuée avec utilisation d'un appareil de rectification à bulles, à des températures au bas de la colonne inférieures à 250 ° C environ.

4. Procédé selon la revendication 1 ou 2, caractérisé en ce que la rectification est effectuée avec utilisation d'un évaporateur à couche mince ou à film tombant, à des températures au bas de la colonne inférieures à 280 ° C environ.

5. Procédé selon une ou plusieurs des revendication 1 à 4, caractérisé en ce que la colonne de séparation présente entre environ 10 et 150 plateaux théoriques.

6. Procédé selon une ou plusieurs des revendication 1 à 5, caractérisé en ce que la rectification est effectuée en présence d'un gaz inerte.

7. Procédé selon une ou plusieurs des revendication 1 à 6, caractérisé en ce que la rectification est effectuée en mode discontinu avec une ou plusieurs colonnes.

8. Procédé selon une ou plusieurs des revendication 1 à 6, caractérisé en ce que la rectification est effectuée en continu avec une ou plusieurs colonnes.

9. Procédé selon une ou plusieurs des revendication 1 à 8, caractérisé en ce que l'on isole l'acide 2-(4-isobutylphényl)-propionique à partir d'un mélange qui est produit lors de la carbonylation du 1-(4-isobutylphényl)-éthanol avec du monoxyde de carbone en présence de triphénylphosphine et éventuellement en présence d'un halognéure de métal de transition.

10. Procédé selon une ou plusieurs des revendication 1 à 9, caractérisé en ce que l'acide 2-(4-isobutylphényl)-propionique est séparé de toutes les impuretés par la rectification.

11. Procédé selon une ou plusieurs des revendication 1 à 9, caractérisé en ce que, pour la rectification, on utilise un mélange qui a été préalablement séparé d'une partie des impuretés par au moins une cristallisation à l'état fondu.

12. Procédé selon une ou plusieurs des revendication 1 à 9, caractérisé en ce que l'on ne sépare par rectification qu'une partie des impuretés, et, pour la séparation des impuretés restantes, on effectue une cristallisation dans des solvants et/ou une cristallisation à l'état fondu.

Fig. 1

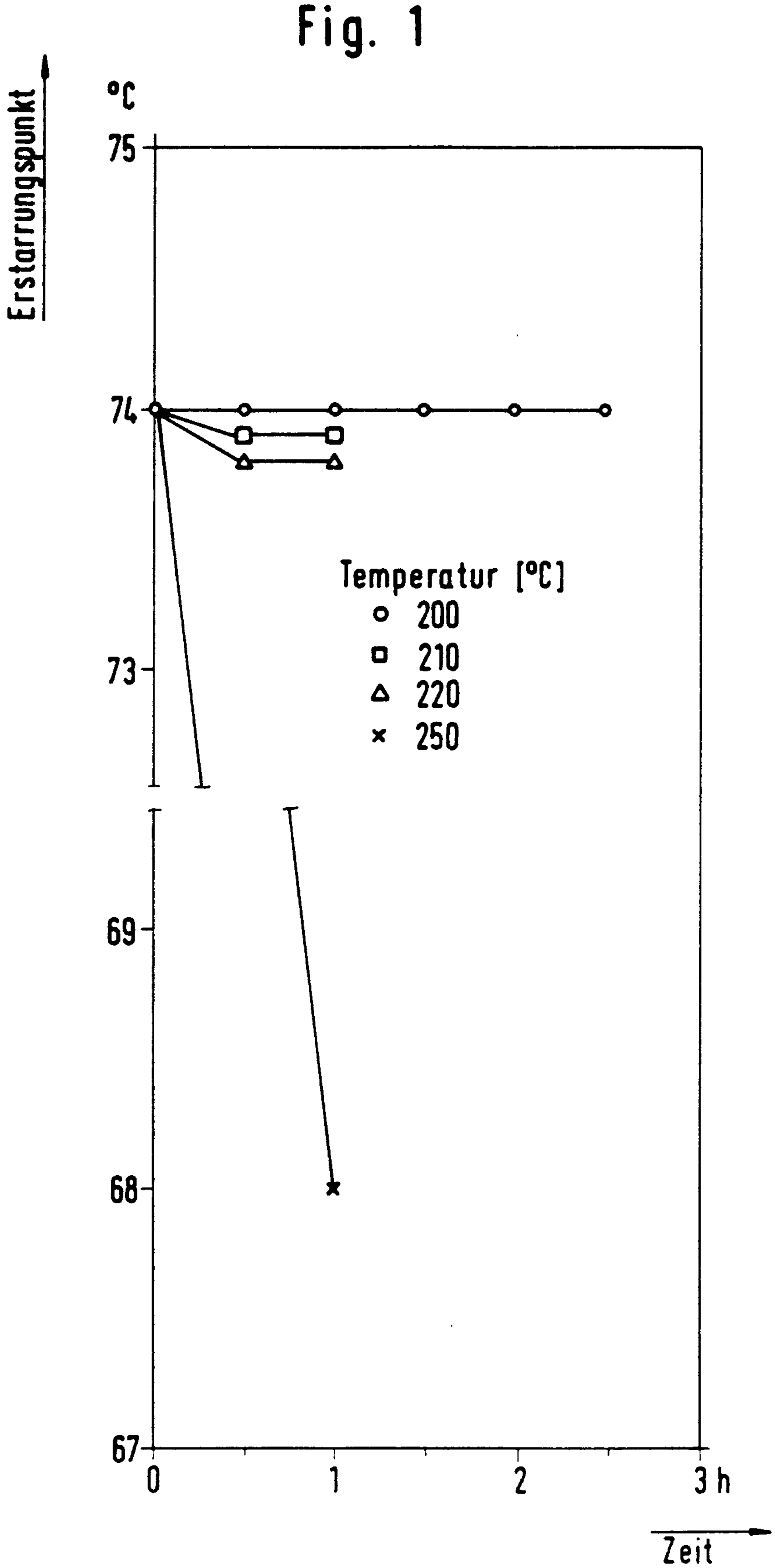
Erstarrungspunkt
°C
75
74
73
69
68
67
0
1
2
3 h
Zeit
Temperatur [°C]
o 200
□ 210
△ 220
× 250

Fig. 2

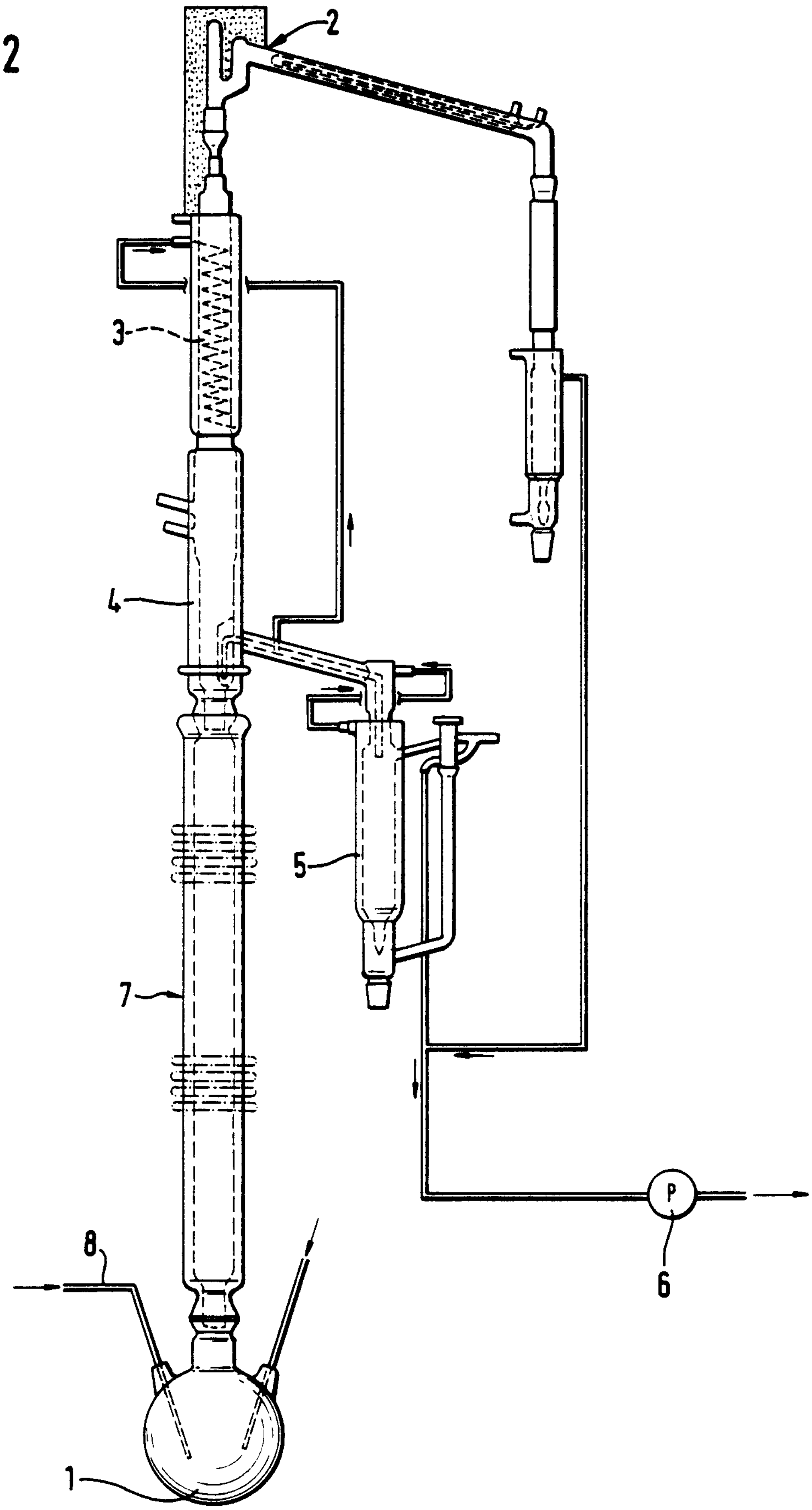

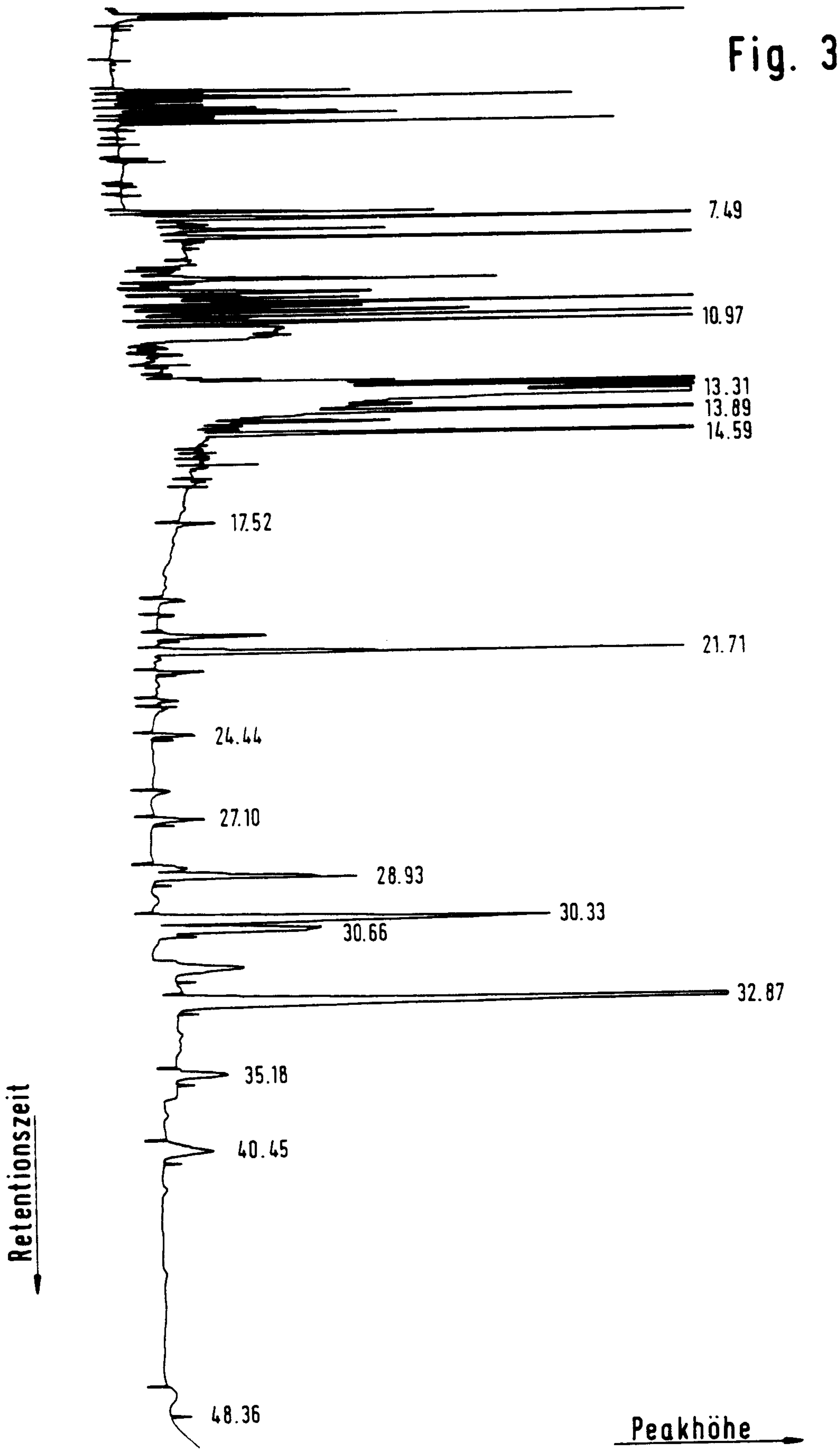
Fig. 3
7.49
10.97
13.31
13.89
14.59
17.52
21.71
24.44
27.10
28.93
30.33
30.66
32.87
35.18
40.45
48.36
Retentionszeit
Peakhöhe

Fig. 4